# EUROPEAN PATENT APPLICATION

(11) **EP 3 804 773 A1**
(43) Date of publication of application: **14.04.2021**
(21) Application number: 19808481.6
(22) Date of filing: 23.05.2019
(51) Int. Cl.: A61L 31/00, A61L 31/04, A61L 31/14

(54) **VISCOELASTIC AGENT MATERIAL**

(30) Priority: 25.05.2018 CN 201810518989; 25.05.2018 CN 201810518788
(71) Applicant: Eyebright Medical Technology (Beijing) Co., Ltd., Beijing 102200 (CN)
(72) Inventor: WEI, Yongji, Beijing 102200 (CN); XIE, Jiangbing, Beijing 102200 (CN)
(74) Representative: Zurhorst, Stefan
(86) International application number: PCT/CN2019/088073
(87) International publication number: WO 2019/223748

(57) **Abstract**

The present invention relates to a viscoelastic agent that is suitable for ophthalmic surgery and can avoid the problem of intraocular lens opacity after ophthalmic surgery (such as cataract surgery), comprising the following substances:
-Viscoelastic substance(s);
-pH buffering agent(s), wherein the pH buffering agent is selected from pH buffering agent(s) based on boric acid and/or borate;
-Osmotic pressure regulator(s);
- Water,
-Optionally, substances with oxidizing and/or reducing properties.

## Description

### TECHNICAL FIELD

The invention is directed to the technical field of biomedical materials, and specifically relates to gel materials used in clinical medical ophthalmology microsurgery, and specifically relates to viscoelastic agent materials suitable for using in ophthalmology surgery.

### BACKGROUND OF THE INVENTION

Cataract is the eye disease with the highest probability of blindness in the world, leading to a significant decrease in term of the vision and quality of life of patients. At present, the only effective method to treat cataract is surgery. After years of development, phacoemulsification cataract extraction technology combined with intraocular lens implantation has become the most commonly used cataract surgery method. As known, any operation will cause damage to the body's cell. In order to improve the quality of the operation, create the operation space, stabilize the intraocular pressure, and reduce the damage to the corneal endothelial cells from the operation, the viscoelastic agents are often used as auxiliary materials for ophthalmic surgery to provide temporary support of the operating space, which protects the tissue from mechanical damage by surgical instruments and facilitates the smooth implementation of the operation, especially, during eye surgery, such as cataract surgery.

Some viscoelastic agents that can be used for ophthalmic surgery purposes are known, for example, viscoelastic agents containing sodium hyaluronate and chondroitin sulfate, such as Alcon Surgical (Alcon Surgical, Inc.) under the name of VISCOAT®. All these highly purified products can be used in specific eye surgery procedures, such as cataract surgery. Due to the following properties of the viscoelastic agent, ophthalmologists use them for several surgical purposes, including maintaining intraocular space and protecting eye tissues, especially corneal endothelial cells, and as an auxiliary manner to manipulate eye tissue. These viscoelastic agents are usually viscous enough to allow surgeons to use them for their intended surgical purposes, but not so viscous that the viscoelastic agents can pass through devices of acceptable internal diameter and be applied to the desired site. However, there is no one viscoelastic agent that can completely satisfy all surgical purposes. For example, in cataract surgery, a combination of relatively low molecular weight sodium hyaluronate and chondroitin sulfate and a phosphate buffer system of viscoelastic agents are very well used to maintain the anterior chamber at any time during cataract surgery, as well as adhere and protects tissues, especially corneal endothelial cells, and is usually called a dispersived viscoelastic agent. However, due to its adhesive properties, the composition of sodium hyaluronate with relatively low molecular weight and chondroitin sulfate is more difficult to remove from the anterior chamber of the eye by suction. On the other hand, viscoelastic agent solutions including sodium hyaluronate with relatively high molecular weight and phosphate buffer systems, such as Healon or PROVISC TM products (Alcon Laboratories, Inc.) are highly cohesive, but cannot have a good adhesion to tissues that may be contacted during surgery, their cohesion and poor adhesion also make them easier to remove from the eye at the end of the operation, but cannot provides good protection to the corneal endothelium after the phacoemulsification of the intraocular lens, wherein it is usually called a cohesive viscoelastic agent.

In addition, phosphate is usually used as a buffer system in viscoelastic agents. Moreover, it is known that viscoelastic agents have many defects in use, such as the opacity of the intraocular lens after cataract surgery. The disclosures as cited by Huang Xin et al. "Research Progress of intraocular lens opacity after cataract surgery" (Huang Xin et al. "Research Progress of Post-cataract IOL Opacity", Chinese Journal of Practical Ophthalmology, Volume 32, Issue 5, May 2014). This article shows that the postoperative opacity rate of the intraocular lens is very high. The opiating intraocular lens is removed by intraocular lens removal process, which causes serious secondary injury to the patient. Moreover, the implantation time is longer, the fusion between the intraocular lens and the human eye tissue is better such that the removal surgery causes greater damages. Therefore, there is a need to develop a new type of viscoelastic agent at this stage, which has the characteristics (such as biosafety, biocompatibility, and has the function of filling, supporting, lubricating and protecting eye tissues such as corneal endothelium), and does not have the defects of the known viscoelastic agents in the prior art (such as potential risks of opacity of the intraocular lens after cataract surgery).

### DISCLOSURE OF THE INVENTION

In order to obtain a viscoelastic agent that has the characteristics (such as biosafety, biocompatibility, and filling, supporting, lubricating and protecting ocular tissues such as corneal endothelium, etc.) required for eye surgery such as humans, and provide viscoelastic agents without existing defects of viscoelastic agents in prior art (such as potential risks that cause intraocular lens opacity after cataract surgery), the present invention provides a viscoelastic agent, which mainly includes a viscoelastic substance(s); pH buffering agent(s), wherein the pH buffering agent(s) may be selected from pH buffering agent(s) based on boric acid and/or borate salt; osmotic pressure regulator(s); and water, optionally substance(s) having oxidizing and/or reducing properties.

The inventor of the present application has surprisingly discovered through research that the use of the viscoelastic agent(s) described in the present application can avoid the problem of intraocular lens opacity after ophthalmic surgery (such as cataract surgery), and has biological safety, biocompatibility, and satisfy the characteristics required for eye surgery such as human eye surgery (such as biosafety, biocompatibility, and has the function of filling, supporting, lubricating and protecting eye tissues such as corneal endothelium).

Regarding the viscoelastic substance, the viscoelastic agent in the present application can include any viscoelastic substances for biomedicine with properties that meet the requirements for eye surgery such as humans eye surgery (such as biosafety, biocompatibility, and has the function of filling, supporting, lubricating and protecting eye tissues such as corneal endothelium) . Preferably, the viscoelastic substance may be selected from: sodium hyaluronate, smart hydrogel, chondroitin sulfate, carboxymethylcellulose sodium and its salts, medical chitosan, collagen, and polypeptides.

The viscoelastic agent of the present invention may also contain therapeutic drugs, which may prevent and treat anterior capsule opacification, posterior capsule opacification, and postoperative intraocular inflammation etc.. Among them, the drugs include various antibiotics, steroidal anti-inflammatory drugs and non-steroidal anti-inflammatory drugs and drugs that prevent postoperative inflammation and microbial infections. The viscoelastic agent of the present invention may also carry anti-metabolic drugs and mitosis inhibitors, immunotoxins and cytotoxins as drugs that inhibit inflammation, drugs that inhibit cell adhesion to extracellular matrix, and drugs that induce apoptosis to eliminate LEC or inhibit mitosis of epithelial cell to prevent and treat posterior capsule opacification, for examples, such as ofloxacin, aspirin, colchicine, lidocaine, nepafenac, ketorolac, bromfenac, recombinant hirudin, methotrexate, 5-fluorouracil, paclitaxel, doxorubicin, daunorubicin, saporin, and other medicines or compositions with similar functions. According to another embodiment of the present invention, the viscoelastic agent further includes other drugs related to treatment(s), wherein the treatment related drugs are based on the final purpose, such as hemostatic drugs, fluorescent agents, photosensitizers, etc. required for surgery.

Regarding the viscoelastic substance, the viscoelastic agent in the present application may include any biomedical viscoelastic substance that meets the characteristics required for eye surgery such as human eye surgery (such as biosafety, biocompatibility, and the function of filling, supporting, lubricating). Preferably, the viscoelastic substance may be selected from: sodium hyaluronate, smart hydrogel, chondroitin sulfate, hydroxypropylmethyl cellulose and its salts, medical chitosan, collagen, and polypeptide.

Regarding pH buffers, the prior art (for example, commercially available viscoelastic agents such as Healon, Amvisc, Amvisc Plus, Previscai, DisCoVisc D, Viscoat) all uses phosphate buffer system(s), wherein there is no viscoelastic agents using other buffer systems in prior art. However, the inventors found that the pH buffering agent that may be used in the present invention may be selected from pH buffering agent(s) based on boric acid and/or borate. Among them, according to the prior art in this field, there are two questions on risks as for the application of pH buffering agent(s) based on boric acid and/or borate as a buffer system to viscoelastic agents used in ophthalmology: whether or not pH buffering system based on boric acid and/or borate may destroy the intermolecular forces of viscoelastic substances such as sodium hyaluronate, change its viscoelasticity, reduce its heat resistance and stability; in addition, whether or not it may cause toxic and inflammatory side effect. Although pH buffers based on boric acid and/or borate are often used in eye drops, such as Naphazoline Hydrochloride and Pheniramine Maleate Eye Drops (Runjie), Siwei zhenpianbing boron eye drops (Zhen Shi Ming), Compound Chondroitin Sulfate Eye Drops (runjie), chloramphenicol eye drops (ruimoushu), XiongDanDiYanYe(RuiHui), Naphazoline Hydrochloride Chlorphenamine Maleate and Vitamin B12 Eye Drops (zhenshiliang). However, the eye drops are substances used on the ocular surface, which are different from viscoelastic agents entering into the eyes, wherein the viscoelastic agents entering into the eyes has a risk resulted by entering into the eyes of viscoelastic agents. In addition, the eye drops including pH buffers based on boric acid and/or borate used in the prior art basically do not need to consider the problem on viscoelastic (rheological) property of the eye drops. If necessary, the adjustable range of viscoelastic (rheological) property is also very large. And there is only one need for the eye drops used on the ocular surface in the prior art, which is to provide partial viscoelasticity. The viscoelastic agent used in ophthalmic surgery not only needs to use its property of support to provide space for surgical operations, but also needs to be easily coated on the surface of surgical instruments and intraocular tissues so as to provide surgical protection. Therefore, it needs to have all the properties required for the viscoelastic agent used for ophthalmic surgery as mentioned above (for example, need to have both cohesion and dispersibility), especially not to deteriorate or even improve the viscoelastic property of viscoelastic agents. Therefore, when considering the buffer system, it must be considered that the viscoelastic property of the original viscoelastic substance cannot be drastically changed, which is very important for the viscoelastic agent of the present invention. Because of its structure of boric acid 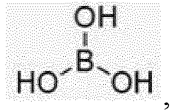 boric acid is very easy to form hydrogen bonds, and compete with the hydrogen bonds themselves of viscoelastic substances, theoretically reduce the viscoelastic property of viscoelastic substances (such as sodium hyaluronate HA). This is the reason why those skilled in the art do not consider using pH buffers based on boric acid and/or borate in viscoelastic agents.

The inventors of the present application have surprisingly discovered after researches that the viscoelastic agents described in the present application including pH buffering agents based on boric acid and/or borate may also obtain superior desired viscoelastic properties and are suitable for the implants used in ophthalmic surgery, as well as have acceptable biological safety.

The inventors of the present application have discovered that the pH buffering agent based on boric acid and/or borate of the present application is capable of providing excellent pH buffering performance while maintaining excellent viscoelastic properties, for example, at a concentration ratio according to the present invention. It avoids the inflammatory reaction caused by viscoelastic agents including the phosphate buffer system, and has passed the rabbit eye implant test to verify its biological safety, and it may be used in, for example, implants for human eye surgery.

The substances with oxidizing and/or reducing properties in the present application may be selected from oxidized glutathione and its salts, reduced glutathione and its salts, sulfur-containing compounds of cysteine sources, proteins containing cysteine/cystine , vitamin B, vitamin C and folic acid.

The inventor also found that, further, the viscoelastic agent in the present application may be added with substances with oxidizing and/or reducing properties suitable for ophthalmic use, such as glutathione etc.. In terms of the properties of ophthalmic viscoelastic agents, viscoelastic agents must maintain a certain degree of cohesion in order to play a supporting role during the operation and provide operating space; but they must also have a certain dispersibility to give the viscoelastic agents excellent a coating performance, protect the eye tissue. However, the addition of substances with oxidizing and/or reducing properties, such as glutathione, will destroy the three-dimensional network system of the original viscoelastic agent gel system due to the change of the intermolecular force, result in severe changes of the elasticity and viscosity of the gel, or even result in physical degradation, so that the viscoelastic agent loses its original properties, thereby loses the role that the viscoelastic agent should play in surgery. This is the reason why substances with oxidizing and/or reducing properties suitable for ophthalmic use, such as glutathione, have been used in eye drops and ophthalmic perfusate, but there is no prior art that uses (or report) substances with oxidizing and/or reducing properties in ophthalmic viscoelastic agents.

The inventors of the present application found that adding substances with oxidizing and/or reducing properties (such as reduced glutathione) with an appropriate amount has weaker competition on the cross-linking points of the three-dimensional network of the gel, and due to the reducibility of viscoelastic substances (such as sodium hyaluronate) and the fixing effect of the three-dimensional gel network on reducing substances, the stability of substances with oxidation and/or reduction properties (such as reduced glutathione) is maintained. Similarly, the addition of substances with oxidizing and/or reducing properties (such as oxidized glutathione) with an appropriate amount may penetrate into the tissue to participate in the conversion into reduced glutathione, thereby improve the scavenging ability of free radicals from tissue and increase the body's antioxidant capacity. The addition of substances with oxidizing and/or reducing properties (such as oxidized glutathione) has a severe effect on the elasticity and viscosity of viscoelastic substances. After further adding the substances with reducing properties (such as vitamin C or folic acid), reduction substances such as reduced glutathione may be obtained, thereby also maintain the cohesion of viscoelastic substances in the viscoelastic agent such as sodium hyaluronate gel. The substances with oxidizing and/or reducing properties such as glutathione have a significant protective effect on corneal endothelial cells due to its hydration in the cornea and the ability to resist oxidative damage etc..

Moreover, the applicant surprisingly found that the introduction of substances with oxidizing and/or reducing properties suitable for ophthalmology, such as glutathione, into the viscoelastic system, on the one hand, may protect corneal cells through redox effects; more importantly, the use of substances with oxidizing and/or reducing properties such as glutathione, which have a low isoelectric point and are suitable for ophthalmology, makes it automatically dissociate into negative charges under physiological conditions, and neutralizes the positive charge generated by collision, friction, vibration caused by using surgical instruments during surgery etc., is compatible with devices and implants with positive charges, reduces mechanical damage to cells, and enable make viscoelastic agents exert better protective properties. Moreover, the viscoelastic agent of the present invention comprising substances with oxidizing and/or reducing properties, such as glutathione, etc., has the properties required for eye surgery such as human eye surgery (such as biosafety, biocompatibility, and the function of filling, supporting, lubricating and protecting eye tissues such as corneal endothelium), and does not exhibit the defects of viscoelastic agents in the prior art (such as the potential risk of causing intraocular lens opacity after cataract surgery).

The viscoelastic agent further comprising oxidation and/or reduction properties disclosed in the present invention thus may provide medical viscoelastic agents of cohesion and/or dispersibility type that have corneal protection function, especially suitable for protecting the tissues in the eye during ophthalmology such as cataract surgery and have the function of facilitating operation.

The viscoelastic agent of the present invention, because the pH buffering agent such as boric acid and/or borate may bind to the amino group in the bacterial protein, may inhibit a variety of bacteria and molds, thereby improve the storage and transportation performance of the viscoelastic agent, improve the storage stability of the viscoelastic agent. Because of viscoelastic substances, such as sodium hyaluronate, which is a mucopolysaccharide, and is a good culture medium for bacteria. Traditional sodium hyaluronate gel requires storage at 2-8°C, otherwise it is extremely prone to deterioration, which makes the storage and transportation requirements be strict and difficult to ensure. However, after using a pH buffering agent based on boric acid and/or borate, the viscoelastic agent has a bacteriostatic function while obtaining improved stability during storage and transportation.

In another aspect, the viscoelastic agent of the present invention optimizes the protective function of the corneal endothelium of the perfusion fluid in cataract surgery. The perfusion fluid used in cataract surgery may maintain cell function and protect the corneal endothelium. However, the viscoelastic agent comprising the traditional phosphate system interferes with the role of calcium ions in the perfusion fluid in protecting corneal endothelial cells; and the viscoelastic agent comprising the traditional phosphate system is difficult to completely protect the corneal endothelial cells. Replacement by a pH buffer based on boric acid and/or borate improves the effectiveness of the perfusate in protecting corneal endothelial cells.

### Detailed description of the invention

The present invention relates to a viscoelastic agent with oxidizing and/or reducing properties, said viscoelastic agent comprising the following components:
- Viscoelastic substances,
- pH buffer, which may be selected from pH buffers based on boric acid and/or borate,
- Osmotic pressure regulator, and
- Water,
- Optionally, substances with oxidizing and/or reducing properties.

According to another embodiment of the present invention, wherein the viscoelastic substance is selected from sodium hyaluronate, smart hydrogel, chondroitin sulfate, hydroxypropyl methylcellulose and its salts, medical chitosan, collagen and polypeptide .

According to another embodiment of the present invention, wherein the viscoelastic substance further includes other viscoelastic substances selected from hydroxypropyl methylcellulose and its salts (e.g. carboxymethylcellulose sodium) and collagen.

According to another embodiment of the present invention, wherein the viscoelastic agent further comprises treatment-related drugs, wherein the treatment-related drugs are based on the final purpose, such as anti-inflammatory drugs, sterilizing drugs, hemostatic drugs, fluorescent agents, photosensitizers, and the like.

According to another embodiment of the present invention, the content of the viscoelastic substance ranges from 0.1% to 4% w/v, preferably 1.0% to 3.5% w/v, especially 1.5% to 2.5% w/v, especially 1.5% 2.2% w/v, more preferably 1.7-2.0% w/v, relative to the total volume of the viscoelastic agent.

According to another embodiment of the present invention, wherein the viscoelastic substance is selected from sodium hyaluronate, preferably its molecular weight being 500 000 to 4000 000, preferably 1000 000 to 4000 000, preferably the molecular weight being 1500 000 to 4000 000, more preferably 2000 000 to 4000 000.

According to another embodiment of the present invention, wherein the viscoelastic substance is a smart hydrogel selected from: polyethylene glycol, polyacrylic acid, poly-N-isopropylacrylamide, poloxamer, chitosan and polyhydroxy compound complex gel. The smart hydrogel is a temperature-sensitive hydrogel, which is based on the hydrophilic-hydrophobic balance of the polymer chain. Under the stimulation of temperature's change, the hydrophobicity of the physical cross-linking area of the gel is changed to present a sol-gel (sol-gel) reversible transformation. Specifically, it becomes in a sol state in an *in vitro* environment. After implantation in the human eye, it forms a gel at the temperature of the human eye; and has a strong supporting force.

According to another embodiment of the present invention, wherein the viscoelastic substance is a polypeptide, such as sodium polyglutamate, polylysine, polyaspartic acid.

According to another embodiment of the present invention, wherein the pH buffering agent further comprises sodium hydroxide.

According to another embodiment of the present invention, wherein the pH buffering agent based on boric acid and/or borate is selected from boric acid and borax, boric acid and sodium hydroxide, borax and hydrochloric acid.

According to another embodiment of the present invention, wherein the pH buffering agent based on boric acid and/or borate is selected from boric acid and/or borax.

According to another embodiment of the present invention, wherein the molar concentration of the boron element in the pH buffering agent based on boric acid and/or borate is not higher than 0.44 mol/L, preferably the molar concentration of the boron element being not higher than 0.34 mol/L, more preferred the molar concentration of the boron element being not higher than 0.24 mol/L, and even not higher than 0.20 mol/L. In particular, the amount of the pH buffering agent is such that the pH of the viscoelastic agent is controlled between 5.5 and 8.5, preferably between 6.8 and 7.6.

According to another embodiment of the present invention, the content of boric acid in the pH buffering agent based on boric acid and/or borate is 0.5%-1.0% w/v, and the preferred content of boric acid is 0.6%-0.9% w/v, more preferably 0.7%-0.85% w/v, relative to the total volume of the viscoelastic agent. According to another embodiment of the present invention, the borax content in the pH buffering agent based on boric acid and/or borate is 0.02%-0.1% w/v, and the preferred borax content is 0.03%-0.09% w/v, and more preferably 0.04%-0.05% w/v, even 0.05%-0.07% w/v, which is relative to the total volume of the viscoelastic agent.

According to another embodiment of the present invention, wherein the osmotic pressure regulator is selected from sodium chloride, potassium chloride, magnesium chloride, glucose and calcium chloride.

According to another embodiment of the present invention, wherein the osmotic pressure regulator is sodium chloride.

According to another embodiment of the present invention, wherein the osmotic pressure regulator is sodium chloride, and its content may be 0.3%-0.7% w/v, 0.3%-0.6% w/v, or even 0.3%-0.5% w /v, or 0.4%-0.5% w/v.

According to another embodiment of the present invention, the viscosity of the viscoelastic agent after sterilization at a shear rate of 0.25 s⁻¹ is greater than 50 Pa·s, particularly 50-300 Pa·s.

According to another embodiment of the present invention, wherein the pH range of the viscoelastic agent is 5.5-8.5, preferably 6.8-7.6.

According to an embodiment of the present invention, wherein the substance with oxidizing and/or reducing properties is selected from the group consisting of oxidized glutathione GSSG and its salts, reduced glutathione (Glutathione) and its salts, sulfur-containing compounds of cysteine sources, proteins containing cysteine/cystine , vitamin B, and mixture thereof.

According to another embodiment of the present invention, wherein the viscoelastic agent further comprises a substance having reducing properties that is compatible with substances having oxidizing and/or reducing properties, such as vitamin C or folic acid.

According to another embodiment of the present invention, wherein the amount of the viscoelastic substance is at least 0.1% by weight, preferably at least 1% by weight, and not more than 20% by weight, preferably not more than 10% by weight, or in particular, not more than 4% by weight, based on the total weight of the viscoelastic agent.

According to another embodiment of the present invention, wherein the amount of the osmotic pressure regulator is such that the osmotic pressure is controlled at 200-400 mOsm/kg, preferably 260-350 mOsm/kg.

According to another embodiment of the present invention, wherein the amount of the osmotic pressure regulator is 0.001-10% by weight, preferably 0.001-5% by weight, more preferably 0.001-3% by weight, based on the total weight of the viscoelastic agent.

According to another embodiment of the present invention, wherein the amount of the pH buffering agent is the amount such that the pH of the viscoelastic agent (such as a viscoelastic agent with oxidation and/or reduction properties) is controlled at 5.5-8.5, preferably 6.8-7.6.

According to another embodiment of the present invention, wherein the amount of the substance with oxidation and/or reduction properties is 0.0001-10% by weight, preferably 0.001-5% by weight, more preferably 0.001-3% by weight, based on the total weight of the viscoelastic agent.

According to another embodiment of the present invention, the viscoelastic agent comprises the following components or consists of the following components:
- Sodium hyaluronate: 1%-2.5% w/v;
- Boric acid: 0.7%-0.85% w/v;
- Borax: 0.04%-0.07% w/v;
- Sodium chloride: 0.3%-0.7% w/v;
- The balance is water.

According to another embodiment of the present invention, wherein, relative to the total weight of the viscoelastic agent, the viscoelastic agent comprises:
- 1.2-4.8% by weight of sodium hyaluronate,
- 0.0001-1% by weight of oxidized glutathione,
- The balance is pH buffer, osmotic pressure regulator and water with effective amounts.

According to another embodiment of the present invention, wherein, relative to the total weight of the viscoelastic agent, the viscoelastic agent comprises:
- 1.2-4.8% by weight of sodium hyaluronate,
- 0.0001-1% by weight of reduced glutathione,
- The balance is effective amounts of pH buffer, osmotic pressure regulator and water.

According to another embodiment of the present invention, wherein, relative to the total weight of the viscoelastic agent, the viscoelastic agent comprises:
- 1.2-4.8% by weight of sodium hyaluronate,
- 0.0001-1% by weight of reduced glutathione;
- 0.0001-1% by weight of vitamin C;
- The balance is effective amounts of pH buffer, osmotic pressure regulator and water.

According to another embodiment of the present invention, wherein, relative to the total weight of the viscoelastic agent, the viscoelastic agent comprises:
- 1.2-4.8% by weight of sodium hyaluronate,
- 0.0001-1% by weight of oxidized glutathione;
- 0.7-0.85 % by weight of boric acid,
- 0.04-0.07% by weight of borax,
- 0.3-0.7% by weight of sodium chloride,
- The balance is water.

According to another embodiment of the present invention, wherein, relative to the total weight of the viscoelastic agent, the viscoelastic agent comprises:
- 1.2-4.8% by weight of sodium hyaluronate,
- 0.0001-1% by weight of reduced glutathione,
- 0.7-0.85 % by weight of boric acid,
- 0.04-0.07% by weight of borax,
- 0.3-0.7% by weight of sodium chloride,
- The balance is water.

According to another embodiment of the present invention, wherein, relative to the total weight of the viscoelastic agent, the viscoelastic agent comprises:
- 1.2-4.8% by weight of sodium hyaluronate,
- 0.0001-1% by weight of reduced glutathione,
- 0.0001-1% by weight of vitamin C,
- 0.7-0.85 % by weight of boric acid,
- 0.04-0.07% by weight of borax,
- 0.3-0.7% by weight of sodium chloride,
- The balance is water.

The viscoelastic agent of the present invention is a viscoelastic agent suitable for ophthalmology, in particular, a viscoelastic agent suitable for intraocular use in eye.

The present invention also relates to an ophthalmic device, which comprises the above-mentioned viscoelastic agent of the present invention.

The present invention also relates to an ophthalmic implant, which comprises the above-mentioned viscoelastic agent of the present invention.

The present invention also relates to a method for preparing the viscoelastic agent of the present invention, including the following steps:
- Dissolving a pH buffer agent(s) and osmotic pressure regulator, obtaining a sterile buffer,
- Adding the viscoelastic substance to the sterile buffer,
- Optionally adding treatment-related drugs to the viscoelastic agent.

The present process for preparing the viscoelastic agent of the present invention further comprises the following steps:
- Dispersing the substance with oxidizing and/or reducing properties in the viscoelastic substance, preferably by a method selected from chemical grafting, modification, physical mixing or supramolecular action so as to disperse the substance with oxidizing and/or reducing properties in the viscoelastic substance. The present invention also relates to a use of ophthalmic devices or ophthalmic implants including the viscoelastic agent of the present invention in ophthalmic surgery.

According to another embodiment of the present invention, the ophthalmic surgery is selected from: intracapsular cataract extraction, phacoemulsification, keratoplasty, intraocular lens implantation and removal, iridotomy, anterior chamber bleeding operation, foreign body and tumor removal, trabeculectomy, vitrectomy or foreign body removal, retinal detachment and epiretinal resection. According to another aspect of the present invention, the present invention also relates to a use of the viscoelastic agent according to the present invention in the treatment of ophthalmic diseases.

According to another aspect of the present invention, the present invention also relates to a method of using the viscoelastic agent according to the present invention to treat ophthalmic diseases, such as filling the eye.

### Description of the drawings:

Figure 1 is a graph of rheological properties of formulation 1 of Example 1.
Figure 2 is a graph of rheological properties of formulation 2 of Example 1.
Figure 3 is a graph of rheological properties of formulation 3 of Example 1.

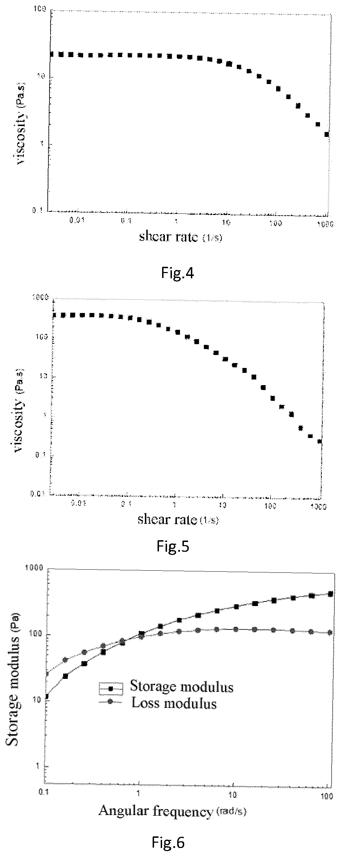

Figure 4 is a graph showing the rheological properties of the viscoelastic agent formed by adding oxidized glutathione in Example 4.
Figure 5 is a graph showing the rheological properties of the viscoelastic agent formed by adding reduced glutathione in Example 6.
Figure 6 is a graph showing the modulus being varied as a function of frequency of the viscoelastic agent formed by adding reduced glutathione in Example 6.

### Examples

Hereinafter, the present invention will be described in more detail through specific examples, but the provided examples are only illustrative and not intended to limit the present invention.

### Example 1

According to Table 1 below, the viscoelastic agents of the present invention having formulations 1-9 were prepared according to the following steps. First, the pH buffer and the osmotic pressure regulator powder were dispersed and dissolved in water for injection, and filtered through aqueous filter membrane with 0.22 µm to obtain a sterile buffer. In a dynamic 100-level environment, added the viscoelastic substance weighed according to the corresponding formula to the sterile buffer, shacked and mixed, and then standed at 2-8°C to remove bubbles. The mixed and bubble-free liquid is filled into the prefilled syringe through the prefilled syringe filling machine under the protection of the oRABS aseptic isolation system, and sterilized by moist heat.

**Table 1**

| | Formula 1 | Formula 2 | Formula 3 | Formula 4 | Formula 5 | Formula 6 | Formula 7 | Formula 8 | Formula 9 |
|---|---|---|---|---|---|---|---|---|---|
| HA (hyaluronic acid) (molecular weight 1.2 million) | 1.0% | - | - | 1.5% | - | - | - | 2.0% | - |
| HA (hyaluronic acid) (molecular weight 1.5 million) | - | - | - | - | - | 1.5% | - | - | - |
| HA (hyaluronic acid) (molecular weight is 2 million) | - | 2.0% | 2.5% | - | 1.7% | - | 1.7% | - | 1.5% |
| CS (chondroitin sulfate) | - | - | - | - | - | 4% | 2% | - | - |
| HPMC (hydroxypropyl methylcellulose) | - | - | - | - | - | - | - | 2% | 3% |
| Boric acid | | 0.75% | 0.76% | 0.650% | - | 0.5% | 0.9% | 0.80% | 0.65% |
| Borax | 0.09% | 0.053% | 0.05% | 0.09% | 0.1% | 0.02% | 0.04% | 0.053% | - |
| Sodium hydroxide | - | - | - | - | - | - | - | - | 0.12% |
| HCl | 0.45% | - | - | - | 0.5% | - | - | - | - |
| Sodium chloride | 0.55% | 0.45% | 0.37% | 0.520% | 0.500% | 0.40% | 0.60% | 0.38% | 0.50% |
| Magnesium chloride | - | - | - | - | - | - | - | - | 0.05% |
| Calcium chloride | - | - | - | - | - | - | - | - | 0.04% |
| Potassium chloride | - | - | - | - | - | - | - | 0.04% | - |
| pH | - | - | - | 7.60 | 7.22 | 7.31 | 7.10 | 6.98 | 7.02 |
| Osmotic pressure | 310 | 298 | 302 | 305 | 300 | 280 | 330 | 287 | 315 |
| Shear viscosity ( 0.25s⁻¹) | 80 | 290 | 430 | 100 | 240 | 200 | 255 | 160 | 252 |

o

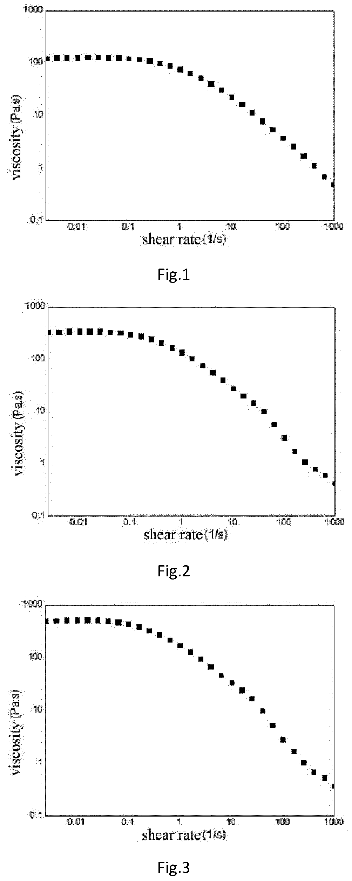

Placed the viscoelastic agent of the above formulae encapsulated in a prefilled syringe in a moist heat sterilization cabinet with pressure compensation, setted the sterilization temperature at 121 °C and F0=8, tested by using a rheometer (Supplier: TA , Model: DHR-1) before and after sterilization. As shown in the above examples and Figures 1-3, the viscoelastic agent of the present invention may withstand moist heat sterilization. After conventional final moist heat sterilization, the viscoelastic agent of the present invention still has excellent viscosity, the viscoelastic agent comprising pH buffering agent based on boric acid and/or borate of the present invention still has the required viscoelastic property and so on.

### Example 2: Rabbit eye implantation test

Before surgery, evaluated and recorded the eyeball by using a rebound tonometer, slit lamp microscope and UBM. Eliminated animals with abnormal eyeballs, chosen 6 groups of control (comparative) eyes and 6 groups of test eyes.

About 25% of the anterior chamber fluid in one eye of the test animal were replaced by using the same amount of the viscoelastic agent Formula 2 of Example 1 of the present invention (the test OVD), and the another eye in other side was operated in the same way with the control OVD. Before starting the next animal, operated on one eyeball and then the other eyeball in a random order. At 24h and 7 days, the verification reactions that occurred during the operation were recorded, as shown in Table 2.

**Table 2**

| Observation item | | Test eye | Control eye |
|---|---|---|---|
| Corneal transparency | 24h | normal | normal |
| | 7d | normal | normal |
| Cells | 24h | normal | normal |
| | 7d | normal | normal |
| Fibrin | 24h | normal | normal |
| | 7d | normal | normal |
| Tyndall (flash) | 24h | Non | Non |
| | 7d | Non | Non |
| Iritis | 24h | Non | Non |
| | 7d | Non | Non |
| Transparency of intraocular lens | 24h | normal | normal |
| | 7d | normal | normal |

The viscoelastic agent of the present invention passed the rabbit eye implantation test and verified its biological safety. There were no differences in rabbit corneal transparency, transparency of intraocular lens, cells number, etc. between before and after implantation, and there was no anterior chamber flash (Tyndall), iritis, and proliferation of fibrin etc., or complications, and the viscoelastic agent of the present invention had good biological safety.

### Example 3 Rabbit eye cell endothelial technology test

The 6 groups of test groups underwent phacoemulsification and lens implantation, a 3mm transparent corneal tunnel incision was made by cutting, and 0.2ml of the viscoelastic agent of Example 1 of the present invention (after moist heat sterilization) was injected through the anterior chamber, and continuous circular capsulorhexis and then ultrasonic emulsified, and aspirated the cortex. The folded intraocular lens was placed in the capsular bag, and applied anti-inflammatory syrup after the operation. The 6 groups of Control groups 1 were subjected to the same operations, and the phosphate pH buffering agent was used by replacing the pH buffering agent in the viscoelastic agent of the formulation 2 of Example 1 of the present invention. The 6 groups of Control groups 2 were subjected to the same operation replaced the viscoelastic agent with a pH buffer of sodium dihydrogen phosphate, so that the pH of the viscoelastic agent was 6.9. The density of corneal endothelial cells was checked by non-contact corneal endothelial microscope before and after surgery, and the average loss rate of corneal endothelial cells was calculated.

**Table 3**

| Observation items | Test group | Control group 1 | Control group 2 |
|---|---|---|---|
| the average loss rate of corneal endothelial cells before and after surgery (%) | 2% | 4.9% | 4% |

The viscoelastic agent of the present invention had a higher protective effect on the corneal endothelium than that of the viscoelastic agent comprising phosphate system. The loss rate of corneal endothelial cells before and after surgery was small, and the viscoelastic agent of the present invention had an excellent protective effect on corneal endothelial cells.

### Example 4:

Weighted 0.0092g of oxidized glutathione, 0.75g of boric acid, 0.053g of borax, 0.4g of sodium chloride, then added appropriate amount of water for injection to dissolve them, added 3.0g of dry sodium hyaluronate powder into the above, added water for injection to make up the weight to 100g, placed a shaker and stirred thoroughly until uniform, formulated concentration of sodium hyaluronate solution was 3.0%, and formulated the concentration of oxidized glutathione solution was 0.15 mmol/L, left to stand at a low temperature and sterilized under high temperature and high pressure at 121 °C for 12 minutes.

### Example 5:

Weighted 0.0092g of oxidized glutathione, 0.01g of vitamin C, 0.75g of boric acid, 0.053g of borax, 0.4g of sodium chloride, added appropriate amount of water for injection to dissolve them, then added 3.4g of dry sodium hyaluronate powder into the above, added water for injection to make up the weight to 100g, placed a shaker and stirred thoroughly until uniform, formulated concentration of sodium hyaluronate solution was 3.0%, formulated concentration of oxidized glutathione solution was 0.15 mmol/L, and formulated concentration of vitamin C solution was 0.01%, left to stand at a low temperature and sterilized under high temperature and high pressure at 121°C for 12 minutes.

### Example 6:

Weighted 0.0092g of reduced glutathione, 0.75g of boric acid, 0.053g of borax, 0.4g of sodium chloride, added appropriate amount of water for injection to dissolve them, then added 2.0g of dry sodium hyaluronate powder into the above, added water for injection to make up the weight to 100g, placed a shaker and stirred thoroughly until uniform, formulated concentration of sodium hyaluronate solution was 2.0%, formulated concentration of reduced glutathione solution was 0.3 mmol/L, left to stand at a low temperature and sterilized under high temperature and high pressure at 121°C for 12 minutes.

### Example 7:

Weighted 0.0092g of reduced glutathione, 0.01g of vitamin C, 0.75g of boric acid, 0.053g of borax, 0.4g of sodium chloride, added appropriate amount of water for injection to dissolve them, then added 2.4g of dry sodium hyaluronate powder into the above, added water for injection to make up the weight to 100g, placed a shaker and stirred thoroughly until uniform, formulated concentration of sodium hyaluronate solution was 2.4%, formulated concentration of reduced glutathione solution was 0.3 mmol/L, and formulated concentration of vitamin C solution was 0.01%, left to stand at a low temperature and sterilized under high temperature and high pressure at 121 °C for 12 minutes.

### Example 8: Rabbit eye implantation test

Before surgery, evaluated and recorded the eyeball by using a rebound tonometer, slit lamp microscope and UBM, eliminated animals with abnormal eyeballs, chosen 6 groups of control eyes and 6 groups of test eyes.

About 25% of the anterior chamber fluid in one eye of the test animal was replaced by using the same amount of the viscoelastic agent of Example 4 of the present invention, and the another eye in other side was operated in the same way with the control OVD. Before starting the next animal, operated on one eyeball and then the other eyeball in a random order. At 24h and 7 days, the verification reactions that occurred during the operation were recorded, as shown in Table 4.

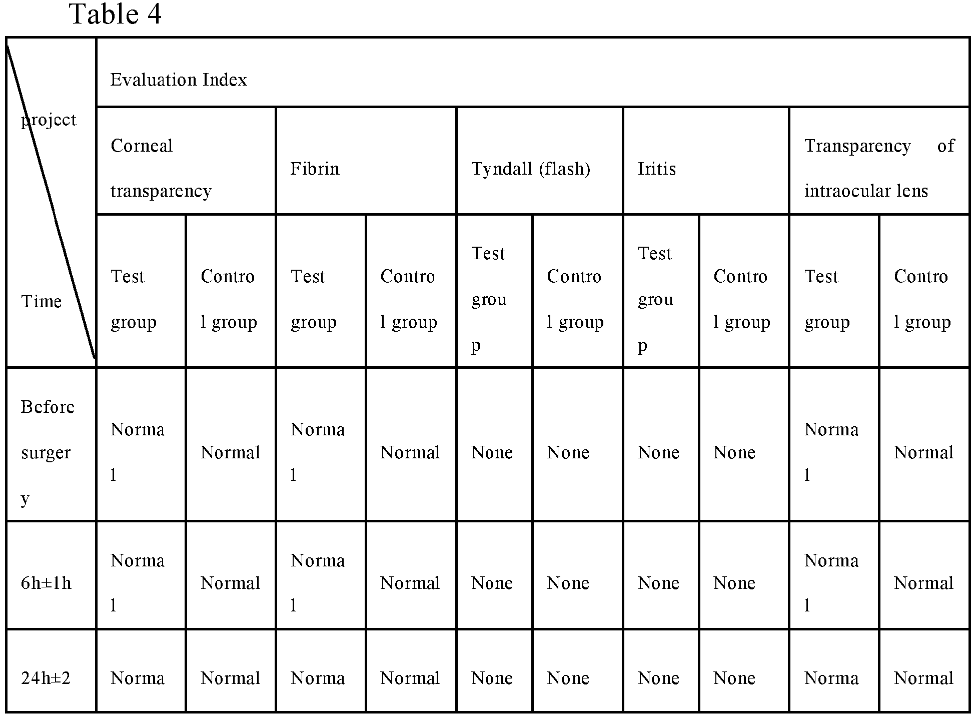

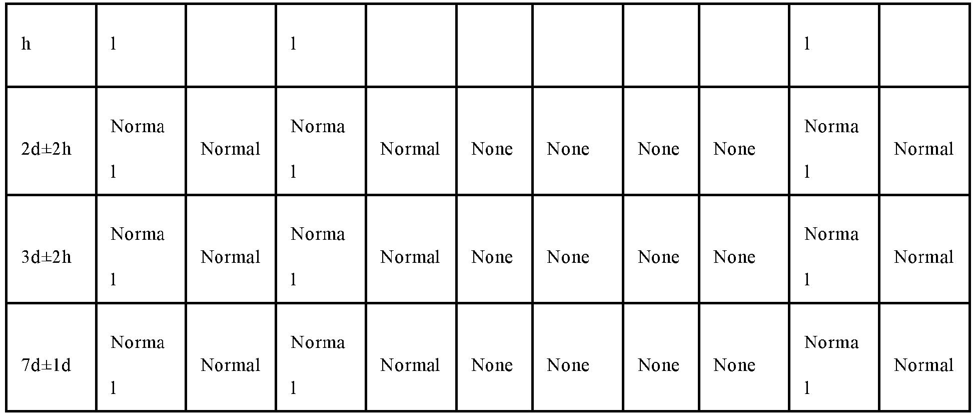

The viscoelastic agent of the present invention passed the rabbit eye implantation test and verified its biological safety. There were no differences in rabbit corneal transparency, transparency of intraocular lens, cells number, etc. between before and after implantation, and there was no anterior chamber flash (Tyndall), iritis, and proliferation of fibrin etc., or complications, and the viscoelastic agent of the present invention had good biological safety.

### Example 9: Rabbit eye cell endothelial technology test

The 6 groups of test groups underwent phacoemulsification and lens implantation, a 3mm transparent corneal tunnel incision was made by cutting, and 0.2ml of the viscoelastic agent of Example 1 of the present invention (after moist heat sterilization) was injected through the anterior chamber, and continuous circular capsulorhexis and then ultrasonic emulsified, and aspirated the cortex. The folded intraocular lens was placed in the capsular bag, and applied anti-inflammatory syrup after the operation. The 6 groups of Control groups 1 were subjected to the same operations, and the phosphate pH buffering agent was used by replacing the pH buffering agent in the viscoelastic agent of Example 7 of the present invention. The 6 groups of Control groups 2 were subjected to the same operation replaced the viscoelastic agent with a pH buffer of sodium dihydrogen phosphate/hydrochloric acid/sodium hydroxide buffer system, so that the pH of the viscoelastic agent was 7.2. The density of corneal endothelial cells was checked by non-contact corneal endothelial microscope before and after surgery, and the average loss rate of corneal endothelial cells was calculated.

**Table 5**

| Observation items | Test group | Control group 1 | Control group 2 |
|---|---|---|---|
| Average loss rate of corneal endothelial cells before and after surgery (%) | 2.45±0.74% | 4.12+1.36% | 4.93±1.92% |

The viscoelastic agent of the present invention had a higher protective effect on the corneal endothelium than that of the viscoelastic agent comprising phosphates system. The loss rate of corneal endothelial cells before and after surgery was small, and the viscoelastic agent of the present invention had an excellent protective effect on corneal endothelial cells.

### Example 10: Rabbit eye cell endothelial technology test

The 6 groups of test groups underwent phacoemulsification and lens implantation, a 3mm transparent corneal tunnel incision was made by cutting, and 0.2ml of the viscoelastic agent of Example 6 of the present invention (after moist heat sterilization) was injected through the anterior chamber, and continuous circular capsulorhexis and then ultrasonic emulsified, and aspirated the cortex. The folded intraocular lens was placed in the capsular bag, and applied anti-inflammatory syrup after the operation. The 6 groups of Control groups 1 were subjected to the same operations, made a control viscoelastic agent by only removing the reduced glutathione from the viscoelastic agent of Example 7 of the present invention. The density of corneal endothelial cells was checked by non-contact corneal endothelial microscope before and after surgery, and the average loss rate of corneal endothelial cells was calculated.

**Table 6**

| Observation items | Test group | Control group 1 |
|---|---|---|
| Average loss rate of corneal endothelial cells | 2.74±0.88% | 4.65±1.05% |
| before and after surgery (%) | | |

o

When the viscoelastic agent of the present invention comprises a substance with oxidizing and/or reducing properties, the viscoelastic agent of the present invention has a higher protective effect on the corneal endothelium than that of a viscoelastic agent that does not comprise the substance with oxidizing and/or reducing properties of the present invention. The loss rate of corneal endothelial cells is small between before and after surgery, and it has excellent protection of corneal endothelial cells.

## Claims

1. Viscoelastic agent, comprising the following substances:
- Viscoelastic substance(s);
- pH buffering agent(s), wherein the pH buffering agent is selected from pH buffering agent(s) based on boric acid and/or borate;
- Osmotic pressure regulator(s);
- Water,
- Optionally, substances with oxidizing and/or reducing properties.

2. The viscoelastic agent according to claim 1, wherein the viscoelastic substance is selected from sodium hyaluronate, smart hydrogel, chondroitin sulfate, hydroxypropyl methylcellulose and its salts, medical chitosan, collagen and peptides.

3. The viscoelastic agent according to claim 1 or 2, wherein the molar concentration of the boron element in the pH buffering agent based on boric acid and/or borate is no higher than 0.44 mol/L, preferably no higher than 0.34 mol/L, more preferred no higher than 0.24 mol/L.

4. The viscoelastic agent according to any one of the preceding claims, wherein the osmotic pressure regulator is selected from sodium chloride, potassium chloride, magnesium chloride and calcium chloride.

5. The viscoelastic agent according to any one of the preceding claims, wherein the substance with oxidizing and/or reducing properties is selected from the group consisting of oxidized glutathione and its salts, reduced glutathione and its salts, sulfur-containing compounds of cysteine sources, proteins containing cysteine/cystine , vitamin B, and mixture thereof.

6. The viscoelastic agent according to any one of the preceding claims, consisting of the following components:
- Sodium hyaluronate: 1%-2.5% w/v;
- Boric acid: 0.7%-0.85% w/v;
- Borax: 0.04%-0.07% w/v;
- Sodium chloride: 0.3%-0.7% w/v;
- The balance is water.

7. An ophthalmic device comprising the viscoelastic agent according to any one of the preceding claims.

8. An ophthalmic implant comprising the viscoelastic agent according to any one of the preceding claims 1 to 6.

9. The process for preparing the viscoelastic agent according to any one of the preceding claims 1 to 6, comprising the following steps:
- Dissolving pH buffer agent(s) and osmotic pressure regulator(s), to obtain a sterile buffer,
- Adding viscoelastic substance to the sterile buffer,
- Optionally, dispersing the substance with oxidizing and/or reducing properties in the viscoelastic substance, preferably by a method selected from chemical grafting, modification, physical mixing or supramolecular action so as to disperse the substance with oxidizing and/or reducing properties in the viscoelastic substance.

10. Use of the viscoelastic agent according to any one of the preceding claims 1 to 6 or the ophthalmic device according to the preceding claim 7 or the ophthalmic implant according to the preceding claim 8 in ophthalmic surgery.
